(19) Europäisches Patentamt
European Patent Office
Office européen des brevets

(11) **EP 2 354 790 B1**

(12) **EUROPEAN PATENT SPECIFICATION**

(45) Date of publication and mention of the grant of the patent:
**17.07.2013 Bulletin 2013/29**

(21) Application number: **09830449.6**

(22) Date of filing: **03.12.2009**

(51) Int Cl.:
***G01N 33/53*** *(2006.01)* ***G01N 27/62*** *(2006.01)*
***G01N 30/88*** *(2006.01)* ***G01N 33/574*** *(2006.01)*
***G01N 33/68*** *(2006.01)*

(86) International application number:
**PCT/JP2009/070311**

(87) International publication number:
**WO 2010/064683 (10.06.2010 Gazette 2010/23)**

(54) **METHOD FOR DETERMINING PROSTATE CANCER**

VERFAHREN ZUM NACHWEIS VON PROSTATAKREBS

PROCÉDÉ POUR DÉTERMINER LE CANCER DE LA PROSTATE

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **03.12.2008 JP 2008309012**

(43) Date of publication of application:
**10.08.2011 Bulletin 2011/32**

(73) Proprietor: **The Noguchi Institute**
**Tokyo 173-0003 (JP)**

(72) Inventors:
- **HIRANO Kiyoko**
  **Tokyo 173-0003 (JP)**
- **NAKAMURA Toshio**
  **Tokyo 173-0003 (JP)**
- **AMANO Junko**
  **Tokyo 173-0003 (JP)**

(74) Representative: **von Kreisler Selting Werner**
**Deichmannhaus am Dom**
**Bahnhofsvorplatz 1**
**50667 Köln (DE)**

(56) References cited:
**JP-A- 2002 055 108 JP-T- 2005 500 057**

- **CHANDRASEKARAN E V ET AL: "The pattern of glycosyl- and sulfotransferase activities in cancer cell lines: a predictor of individual cancer-associated distinct carbohydrate structures for the structural identification of signature glycans", CARBOHYDRATE RESEARCH, PERGAMON, GB, vol. 341, no. 8, 12 June 2006 (2006-06-12) , pages 983-994, XP025010271, ISSN: 0008-6215, DOI: 10.1016/J.CARRES. 2006.02.017 [retrieved on 2006-06-12]**
- **TABARES GLORIA ET AL: "Different glycan structures in prostate-specific antigen from prostate cancer sera in relation to seminal plasma PSA", GLYCOBIOLOGY, OXFORD UNIVERSITY PRESS, US, vol. 16, no. 2, 1 February 2006 (2006-02-01), pages 132-145, XP002582316, ISSN: 0959-6658, DOI: 10.1093/GLYCOB/CWJ042**
- **JANKOVIC M ET AL: "Glycans as a target in the detection of reproductive tract cancers", JMB, vol. 27, no. 1, January 2008 (2008-01), - March 2008 (2008-03), pages 17-19, XP002680795, ISSN: 1452-8258**
- **PERACAULA R ET AL: "Altered Glycosylation Pattern Allows the Distinction Between Prostate-specific Antigen (PSA) from Normal and Tumor Origins", GLYCOBIOLOGY, OXFORD UNIVERSITY PRESS, US, vol. 13, no. 8, 1 June 2003 (2003-06-01), pages 457-470, XP002995528, ISSN: 0959-6658, DOI: 10.1093/GLYCOB/CWG041**
- **CHIKARA OHYAMA ET AL.: 'Carbohydrate structure and differential binding of prostate specific antigen to Maackia amurensis lectin between prostate cancer and benign prostate hypertrophy' GLYCOBIOLOGY vol. 14, no. 8, 2004, pages 671 - 679, XP008139724**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 354 790 B1

- **GLORIA TABARES ET AL.: 'Different glycan structures in prostate-specific antigen from prostate cancer sera in relation to seminal plasma PSA' GLYCOBIOLOGY vol. 16, no. 2, 2006, pages 132 - 145, XP002582316**
- **SHUHEI SUMI ET AL.: 'Serial lectin affinity chromatography demonstrates altered asparagine- linked sugar-chain structures of prostate- specific antigen in human prostate carcinoma' JOURNAL OF CHROMATOGRAPHY B vol. 727, 1999, pages 9 - 14, XP008139533**
- **ROSA PERACAULA ET AL.: 'Altered glycosylation pattern allows the distinction between prostate-specific antigen (PSA) from normal and tumor origins' GLYCOBIOLOGY vol. 13, no. 6, 2003, pages 457 - 470, XP002995528**
- **CHIKARA OYAMA: 'Hinyoki Akusei Shuyo no Tosa Seibutsugaku' JAPANESE JOURNAL OF ELECTROPHORESIS vol. 46, 2002, pages 51 - 54, XP008139956**

**Description**

Technical Field

The present invention relates to a novel method for determining prostate carcinoma, in particular, to a novel method for determining between prostate carcinoma and benign prostatic hyperplasia. Specifically, the present invention relates to a novel method for determining between prostate carcinoma and benign prostate hypertrophy based on the difference in glycan structure of a prostate specific antigen.

Background Art

Prostate carcinoma (hereinafter abbreviated as PC) is one of main common causes of man's death. Prostate specific antigen (hereinafter abbreviated as PSA) is recognized as the most important tumor marker for PC (for example, see Non-Patent Document 1). PSA is a glycoprotein or a derivative thereof, having molecular weight of about 30 kDa and comprised of a protein moiety composed of 237 amino acid residues having molecular weight of about 26 kDa and a glycan moiety bonded to an amino acid residue (such as for example the 45th Asn) of the protein moiety. The glycan moiety occupies about 8% of the PSA's molecular weight.

Benefit of a serum PSA concentration test in the initial PC diagnosis has been already reported in many references; but there is a PSA concentration region, called a gray zone, where either PC or BPH cannot be determined between a man suffered from benign prostatic hyperplasia (hereinafter abbreviated as BPH) and a man suffered from PC (for example, see Non-Patent Document 2).

In order to solve this problem, until now several attempts have been made (for example, determining by PSA density, PSA gradient (annual increase rate), free PSA/total PSA ratio, and so on); but there are considerable overlap between two lesions.

Recently, the result that a glycan structure bonded to PSA's asparagines (N) is different between a PC tissue and a BPH tissue in the research using a continuous lectin affinity chromatography using concanavalin A, plant agglutinin E4 (PHA-E4), and PHA-L4 has been reported (see Non-Patent Document 3). This report describes that an N-bonded glycan in PSA changes in canceration process of human prostate and that the N-bonded glycan in PSA might be useful as a diagnosis tool of PC.

Several immunological methods have been proposed to detect a PSA structure by using a PSA glycan-bonding molecule. For example, a method to distinguish between PC and BPH, wherein PSA is contacted with a lectin so that PSA separated based on the affinity of a PSA glycan with the lectin may be measured, has been reported (see Patent Document 1). In this document, it is described that the difference in affinity of the PSA glycan with the lectin (such as Maackia amurensis lectin) is based on a conformation of sialic acid at a glycan terminal. However, a specific structure of the PSA glycan of a test subject suffered from PC or BPH is not determined.

Alternatively, a method to examine PC based on whether or not a glycan having at least three branches exists in PSA has been reported (see Patent Document 2). In this method, a bonding molecule capable of bonding to a glycan having at least three branches but not to a glycan having one branch and two branches is used. The bonding molecule usable includes a lectin (such as PHA-L) and an antibody.

Alternatively, there is a report focusing on content of N-acetylgalactosamine (GalNAc) in PSA (see Non-Patent Document 4). In the report, structure of a PSA glycan is extensively investigated; and it is described that content of GalNAc in a PSA glycan derived from seminal plasma is 25% while content of GalNAc in a PSA glycan of a prostate carcinoma cell line (LNCaP) is about 65%. Authors of the report found a similar change in a cell line derived from pancreatic carcinoma (Capan-1) (see Non-Patent Document 5), and they assumed from this finding that the change of GalNAc content is caused by cancerous mutation.

Alternatively, a method to comprehensively analyze glycans of a protein object including PSA by using an array comprised of a specific antibody and many lectins has been reported (see Non-Patent Document 6). In this method, it is made clear that there exists a large amount of a LacdiNAc (N-acetylgalactosamine-N-acetylglucosamine) structure in a unreduced terminal of a PSA glycan structure derived from a prostate carcinoma cell line. However, the method does not target a PSA glycan of a test subject suffered from PC or BPH.

In a related matter, it is disclosed that the LacdiNAc structure is an oligo sugar alignment specifically expressed in a carcinoma cell; and a patent is filed on a method to detect a cancer based on its existence detected in a biological sample as the indicator (see Patent Document 3). However, a specific method to detect the existence is not disclosed; and moreover, determining between PC and BPH is not intended.

Further alternatively, a method to evaluate clinical status of a test subject by determining a sugar profile of glycan of a target glycoprotein including PSA has been proposed (see Patent Document 4). In the document, it is described that a PSA glycan of a test subject suffered from PC is different from a normal PSA glycan in analysis using a MALDI-MS method. However, a specific structure of the PSA glycan of the test subject suffered from PC is not described.

As mentioned above, many examples that a PSA glycan structure changes with a disease have been reported; but there are not many detailed reports on a PSA glycan structure of a test subject suffered from BPH, nor the report yet on example of comprehensive analysis of a PSA glycan of a PC patient. On top of that, determining between BPH and PC based on a specific glycan structure has not been done.

Prior Art References

Patent Documents

Patent Document 1: Japanese Patent Laid-Open Publication No. 2002-055108
Patent Document 2: Japanese Patent Application Publication No. 2000-514919
Patent Document 3: Japanese Patent Application Publication No. 2005-500057
Patent Document 4: Japanese Patent Application Publication No. 2006-515927

Non-Patent Documents

Non-Patent Document 1: Stamey, et al., N. Engl. J. Med., 317, 909-916 (1987)
Non-Patent Document 2: Catalona, et al., J. Am. Med. Assoc., 279, 1542-1547 (1998)
Non-Patent Document 3: Sumi, et al., J. Chromatogr. B, 727, 9-14 (1994)
Non-Patent Document 4: Peracaula, et al., Glycobiology, 13(6), 457-470 (2003)
Non-Patent Document 5: Peracaula, et al., Glycobilology, 13(4), 227-244 (2003)
Non-Patent Document 6: Kuno, et al., Mol. Cell Proteomics, 8 (1), 99-108 (2009)
Non-Patent Document 7: Tabares, et al., Glycobilology, 16(2), 132-145 (2006)
Non-Patent Document 8: Bindukumar, et al., J. Chromatogr. B, Analyt. Technol. Biomed. Life Sci., 813(1-2), 113-120 (2004)
Non-Patent Document 9: Zhang, et al., Clin. Chem., 41(11), 1567-1573 (1995)
Non-Patent Document 10: Okada, et al., Biochim. Biophys. Acta, 1525(1-2), 149-160 (2001)

Disclosure of Invention

Problems to be Solved by the Invention

Accordingly, the problems to be solved by the present invention is to provide a method and the like for accurate determining between BPH and PC by detecting a PSA glycan of a test subject, in particular by detecting a PSA glycan of a test subject suffered from BPH or PC, thereby conducting the determining based on the PSA glycan structure.

Means for Solving the Problems

Inventors of the present invention found that, in a PSA glycan, prostate carcinoma could be determined, in particular prostate carcinoma and benign prostatic hyperplasia could be determined, by focus attention of the ratio of a glycan having LacdiNAc to a glycan not having LacdiNAc, not only by existence of the LacdiNAc structure as the indicator; and as a result, the inventors accomplished the present invention.
Namely, the present invention is related to the following methods.

[1] A method for determining prostate carcinoma, wherein
the method includes a step of analyzing a PSA glycan structure in a sample derived from a test subject, and prostate carcinoma is determined in the case that amount of a glycan having LacdiNAc (N-acetylgalactosamine-N-acetylglucosamine) (LacdiNAc(+)) is more than 30% of amount of a glycan not having LacdiNAc but having LacNAc (galacotose-N-acetylglucosamine) (LacdiNAc(-)).
[2] The method for determining prostate carcinoma according to [1], wherein
the method includes a step of analyzing a PSA glycan structure in a sample derived from a test subject,
prostate carcinoma is determined in the case that amount of a glycan having LacdiNAc (N-acetylgalactosamine-N-acetylglucosamine) (LacdiNAc(+)) is more than 30% of amount of a glycan not having LacdiNAc but having LacNAc (galacotose-N-acetylglucosamine) (LacdiNAc(-)), and

benign prostatic hyperplasia is determined in the case of 30% or less.

[3] The method for determining prostate carcinoma according to [1], wherein analysis of the PSA glycan structure is done by a method wherein a lectin is acted on the PSA, by a method wherein an antibody is acted on the PSA, by a method with a high performance liquid chromatography or with a mass spectrometry, or by an analysis means wherein these analysis methods are combined.

[4] The method for determining prostate carcinoma according to [2], wherein analysis of the PSA glycan structure is done by a method wherein a lectin is acted on the PSA, by a method wherein an antibody is acted on the PSA, by a method with a high performance liquid chromatography or with a mass spectrometry, or by an analysis means wherein these analysis methods are combined.

[5] The method for determining prostate carcinoma according to [3], wherein
the method includes:

(1) a step of purifying PSA from a sample derived from a test subject,
(2) a step of preparing a PSA derivative from PSA purified in step (1),
(3) a step of labeling the PSA derivative obtained in step (2), and
(4) a step of analyzing the labeled PSA derivative obtained in step (3) by a mass spectrometry method; wherein

a pair of signals shown by the mass difference of 41 due to presence and absence of a LacdiNAc (N-acetylgalactosamine-N-acetylglucosamine) structure is selected, and
prostate carcinoma is determined in the case that a signal strength derived from the glycan having LacdiNAc (LacdiNAc(+)) is more than 30% of a signal strength derived from the glycan not having LacdiNAc but having LacNAc (galacotose-N-acetylglucosamine) (LacdiNAc(-)).

[6] The method for determining prostate carcinoma according to [4], wherein
the method includes:

(1) a step of purifying PSA from a sample derived from a test subject,
(2) a step of preparing a PSA derivative from PSA purified in step (1),
(3) a step of labeling the PSA derivative obtained in step (2), and
(4) a step of analyzing the labeled PSA derivative obtained in step (3) by a mass spectrometry method; wherein

a pair of signals shown by the mass difference of 41 due to presence and absence of a LacdiNAc (N-acetylgalactosamine-N-acetylglucosamine) structure is selected,
prostate carcinoma is determined in the case that a signal strength derived from the glycan having LacdiNAc (LacdiNAc(+)) is more than 30% of a signal strength derived from the glycan not having LacdiNAc but having LacNAc (galacotose-N-acetylglucosamine) (LacdiNAc(-)), and
benign prostatic hyperplasia is determined in the case of 30% or less.

[7] The method for determining prostate carcinoma according to [5], wherein the PSA derivative prepared in step (2) is a glycan derived from PSA or a glycopeptide derived from PSA.

[8] The method for determining prostate carcinoma according to [6], wherein the PSA derivative prepared in step (2) is a glycan derived from PSA or a glycopeptide derived from PSA.

Advantages of the Invention

According to the present invention, the foregoing problems and subjects can be solved. Namely, a method for accurate determining prostate carcinoma, in particular, for accurate determining between prostate carcinoma and benign prostatic hyperplasia, by detecting a glycan structure of a prostate specific antigen (PSA) thereby conducting the determining based on difference in the structure, can be provided.

Specifically, by adopting the foregoing steps, even for test subjects having roughly the same level of PSA concentration in serum and the like, PC and BPH can be distinguished with high accuracy by measuring percentage R value of signal strength ratio of a series of the LacdiNAc(+) glycan structure and the LacdiNAc(-) glycan structure contained in the PSA glycan. In addition, in both PC and BPH, percentage R value of signal strength ratio of LacdiNAc (+)/LacdiNAc(-) is independent of serum PSA concentration; and because the percentage R value is at least larger than the threshold value R in PC and is equal to or less than the threshold value R in BHP, determining whether PC or not can be made with high accuracy by merely setting the threshold value R independent of the PSA concentration.

Accordingly, for a test subject who shows high PSA value (for example, 4 ng/mL or higher) in the method often used in current PC screening to measure serum PSA concentration and takes a painful needle biopsy as the second examination, an unnecessary examination can be avoided by applying the present invention.

It is known that the serum PSA concentration tends to increase with aging. Accordingly, in an upcoming aging

society in particular, the method of the present invention, which can determine PC or not PC with high accuracy, in particular, which can determine between PC and BPH, will bring about not only medical contribution but also excellent effect in medical economy.

Brief Description of Drawings

[FIG. 1]
FIG. 1 is a chart showing a negative ion MS spectrum obtained in Example 1.
[FIG. 2]
FIG. 2 is a chart showing a negative ion MS/MS spectrum obtained by using the signal $P2_l$ obtained at m/z=2118 as the precursor ion in Example 1.
[FIG. 3]
FIG. 3 is a chart showing a positive ion MS spectrum obtained in Example 4.
[FIG. 4]
FIG. 4 is a chart showing a positive ion MS spectrum obtained in Example 5.
[FIG. 5]
FIG. 5 is a chart showing a positive ion MS spectrum obtained in Example 6.
[FIG. 6]
FIG. 6 is a chart showing a positive ion MS spectrum obtained in Example 7.

Best Modes for Carrying Out the Invention

Hereinbelow, the present invention will be explained, but the present invention is not limited to specific embodiments shown below and can be carried out with any arbitrary modification thereof.

The method of the present invention to determine PC, in particular to determine between PC and BPH, is characterized in that, in a step to analyze an object by a specifically bonding molecule such as a lectin and a specific antibody, by a high performance liquid chromatography, by a mass spectrometry, or by a method using a combination thereof, upon applying a standard threshold value $R(_s)$, prostate carcinoma is determined in the case that, in the detection object, a group containing the LacdiNAc(+) glycan structure is more than 30% of a group containing the LacdiNAc(-) glycan structure, and in particular, benign prostatic hyperplasia is determined in the case of 30% or less.

In the present invention, "N-acetylgalactosamine-N-acetylglucosamine" is abbreviated as "LacdiNAc", and "galactose-N-acetylglucosamine" is abbreviated as "LacNAc". The glycan having LacdiNAc is abbreviated as "LacdiNAc (+)", and the glycan not having LacdiNAc but having LacNAc is abbreviated as "LacdiNAc(-)".
"A sample derived from a test subject" used in the present invention includes a body fluid, such as blood (including serum, plasma, and the like), urine, and sperm (seminal plasma), and a cell and a tissue.

Most preferably, the method of the present invention to determine PC, in particular to determine between PC and BPH is characterized in that, a step of analysis by a mass spectrometry includes:

(1) a step of purifying PSA from a sample derived from a test subject,
(2) a step of preparing a PSA derivative from PSA purified in step (1), and
(3) a step of labeling the PSA derivative obtained in step (2), and then
(4) the labeled PSA derivative obtained in step (3) is analyzed; wherein,

upon applying a standard threshold value $R_{(s)}$,
prostate carcinoma is determined in the case that a signal strength derived from the LacdiNAc(+) glycan structure in the labeled PSA derivative is more than 30% of a signal strength derived from the LacdiNAc(-) glycan structure, and in particular,
not to be prostate carcinoma or to be benign prostatic hyperplasia is determined in the case of 30% or less.

In step (1), PSA is purified from a sample derived from a test subject. Step (1) can be carried out by an arbitrary method known in the art.

Alternatively, in the case that a serum derived from a test subject is used as a sample in step (1), a method with a combination of several kinds of affinity chromatographies may be used. For example, a serum derived from a test subject is purified by Fractogel (registered trade name: EMD TA), Cibacron Blue 3GA, or the like, which are affinity carriers based on sulfurphilic adsorption, or by protein A sepharose CL-4B, HiTrap heparin column HPLC, or the like (see Non-Patent Document 7). The eluted solution thereof is treated with ethanolamine to obtain a free PSA. The obtained PSA is treated with the immunoprecipitation method to obtain a PSA derivative. According to the document, to analyze

the obtained PSA derivative, approximately 63 μg of PSA was necessary in the sample derived from a test subject.

In the case that a sample other than a serum derived from a test subject is used as the sample in step (1), PSA can be purified by using an affinity carrier based on sulfurphilic adsorption in step (1) (see Non-Patent Document 8). For example, PSA is purified from a human sperm, from a serum of a prostate carcinoma patient, or from a supernatant of culture solution of a prostate carcinoma cell (LNCap) by a chromatography using 3S, T-gel slurry (Fractogel (registered trade name) EMD TA); and then PSA itself, or a complex thereof with α1-antichymotrypsin (ACT) or the like, can be identified by using a Western blotting method.

Or alternatively, in the case that a sperm derived from a test subject is used as the sample, PSA may be purified by combination of an affinity chromatography based on sulfurphilic adsorption and gel filtration (see Non-Patent Document 8). For example, a combination of Fractogel (registered trade name) TA 650(S) with gel filtration carrier Ultrogel AcA-54 may be used. With this combined method, a free PSA could be recovered with 72% yield.

Or further alternatively, in the case that a sperm derived from a test subject as the sample in step (1), PSA may be purified by the following method (see Non-Patent Documents 9 and 10). For example, PSA may be purified by sequentially conducting precipitation by addition of ammonium sulfate, a hydrophobic interaction chromatography (Phenyl-Sepharose-HP column), a gel filtration (Sephacryl S-200 column), and an anion-exchange chromatography (Resource Q column). In this method, when the sample contained 33.9 mg of PSA, final recovery yield of PSA was reported to be 30.1%.

In the case that an androgen-dependent LNCaP derived from a test subject is used as the sample in step (1), PSA may be purified by a combination method of ultrafiltration with various kinds of chromatographies (see Non-Patent Document 5). For example, PSA may be purified by sequentially conducting ultrafiltration (polysulfone membrane with 5KDa cut-off (Millipore) of culture solution containing LNCaP cultured by an appropriate means, an affinity chromatography (Cibacron Blue 3GA), a gel filtration (Biogel P60), an affinity chromatography (Cibacron Blue 3GA), and a reversed phase chromatography (214TP-RP C4, using HPLC).

Then, in step (2), a PSA derivative is prepared from the purified PSA. The term "a PSA derivative" in the present invention means a glycan or a glycopeptide derived from PSA; specifically the term means a glycan or a glycopeptide separated from PSA. For example, PSA is treated with an endoprotease (such as thermolysin, endoprotenase Arg-C, endoprotenase Lys-C, trypsin, chymotrypsin, pepsin, proline-specific endopeptidase, protease V8, protenase K, aminopeptidase M, and carboxypeptidase B) or with a breaking agent of a peptide bond thereby forming a glycopeptide, which may be used in step (3).

Alternatively, a free glycan may be formed by treating PSA enzymatically. Further alternatively, a free glycan may be formed by enzymatically treating the foregoing glycopeptide derived from PSA. The glycans thus obtained may be used in step (3). In this enzymatic treatment, for example, peptide N-glycanase (PNGase F and PNGase A), endoglycosidase (EndoH and EndoF), and/or, one or a plurality of proteases (such as trypsin and endoprotenase Lys-C) may be used. Or alternatively, a free glycan may be formed by chemical treatment (such as decomposition by anhydrous hydrazine and a reductive or a non-reductive β-elimination) of PSA or a glycopeptide derived from PSA. The free glycans thus obtained may be used in the following step (3). Alternatively, a glycan or a glycopeptide, obtained by sialidase-treatment or by an acidic hydrolysis to eliminate sialic acid, may be used.

Alternatively, in the case that a PSA band is cut out by an electrophoresis at the final stage of step (1), the cut-out band may be digested in gel by the foregoing reagents so that a glycopeptide and/or a glycan can be formed.

In step (3), the PSA derivative is labeled. As a labeling compound, a compound having a condensed polycyclic hydrocarbon moiety such as naphthalene, anthracene, and pyrene, a reactive functional group capable of making a bond with a molecule to be analyzed, and optionally a spacer moiety that connects between the condensed polycyclic hydrocarbon moiety and the reactive functional group may be used.

The condensed polycyclic hydrocarbon moiety is preferably pyrene. The reactive functional group includes, for example, a diazomethyl group, an amino group, and a hydrazide group, which can react with a carboxy group of sialic acid or a reduced sugar terminal. The spacer moiety includes, for example, a linear or a branched alkylene group. The labeling compound usable in the present invention includes 1-pyrenyl diazomethane (PDAM), 1-pyrene butanoic acid hydrazide (PBH), 1-pyrene acetic acid hydrazide, 1-pyrene propionic acid hydrazide, aminopyrene (including structural isomers), 1-pyrene methylamine, 1-pyrene propylamine, and 1-pyrene butylamine. The most preferably used labeling compound is PDAM.

Step (3) may be conducted by mixing a PSA derivative and a labeling compound, and then heating the resulting mixture. Heating may be conducted in the temperature range, for example, between 40 and 50°C. Optionally and arbitrarily, labeling may be conducted in the presence of an accelerating agent such as a water-soluble carbodiimide and N-hydroxy succinimide. More preferably, labeling may be conducted in such a way that solution of a PSA derivative is dropped onto a target plate used in a MALDI method so as to be dried, and then solution of a labeling compound is dropped onto it so as to be heated and dried.

The present invention relates to a method for analyzing a labeled PSA derivative by mass spectrometry. Hereinafter, "analysis by mass spectrometry" may be abbreviated as "MS analysis". An ionization member usable in MS

analysis in step (4) includes an instrument with a type of matrix-assisted laser desorption ionization (MALDI) and a type of electrospray ionization (ESI).

In the present invention, ionization efficiency of a labeled PSA derivative in a MALDI method can be improved as compared with an unlabeled PSA derivative, by bonding a condensed polycyclic hydrocarbon group such as pyrene. In a labeled PSA derivative of the present invention, an ESI method can be applied more easily. Because, a PSA derivative is highly hydrophilic so that it is difficult to obtain a solution of a sample for an ESI method in an organic solvent; on the other hand, a labeled PSA derivative of the present invention is made soluble in an organic solvent by introduction of the condensed polycyclic hydrocarbon group.

A desorption member usable in MS analysis in step (4) includes any instrument known in the art, such as a time of flight (TOF) type, a double focusing type, and a quadrupole focusing type. In particular, in view of conducting an $MS^n$ ($n \geq 2$) analysis, use of an instrument having an ion trap is convenient. A particularly preferable instrument is a type of quadrupole ion trap-time of flight (QIT-TOF). Here, any of a linear type, a reflectron type, and a multi-turn type may be used as the time of flight instrument.

Then, in the obtained MS spectrum, a pair of signal P1 and signal P2 with the mass difference of 41 due to existence and non-existence of the LacdiNAc structure, corresponding to substitution of one molecule of N-acetylgalactosamine (GalNAc) and galactose (Gal), is selected. Here, P2 means a signal corresponding to the GalNAc-substituted glycan (LacdiNAc(+) glycan) and P1 means a signal corresponding to the GalNAc-unsubstituted glycan (LacdiNAc(-)). Then, the signal strength S (P1) corresponding to the LacdiNAc(-) glycan and the signal strength S (P2) corresponding to the LacdiNAc(+) glycan are measured; and then percentage R value of signal strength ratio is calculated by the following equation. Here, "signal strength" includes strength of, for example, a monoisotopic mass signal, a mass signal with the maximum strength, sum of arbitral selection in a monoisotopic mass signal and an isotope ion signal or an average thereof, in MS spectrum.
Hereinafter, "percentage R value of signal strength ratio" may be abbreviated as merely "R value".

$$\text{R Value} = [S(P2)/S(P1)] \times 100$$

In the case that the standard threshold $R_{(s)}$=30% is used, when the obtained R value is more than 30%, determining that "a test subject is suffered from PC" is made; and when the obtained R value is equal to or less than 30%, determining that "a test subject is not suffered from PC" or "a test subject is suffered from BPH" is made. The threshold R may be set in the range of about 10% above or below of the standard threshold $R_{(s)}$=30% in view of the detection sensitivity due to amount of the determining object and/or the determining accuracy. The threshold R of about 20% is preferable for highly accurate determining; while about 40% is preferable when amount of the determining object is small.

In an MS spectrum obtained in this step, the foregoing determining procedure may be conducted by selecting a plurality of signal pairs having mass difference of 41. Or alternatively, the foregoing determining procedure may be conducted by selecting a pair of signals having mass difference of 41 among the signals caused by release of a labeled compound and/or a sialic acid residue and the like in a mass spectrometer.

The present invention is a method for determining of prostate carcinoma by analyzing a PSA glycan structure in a sample derived from a test subject with a mass spectrometry method, as mentioned above; and at the same time the present invention is a method for determining of prostate carcinoma by analyzing the PSA glycan structure by an analysis method wherein a lectin is acted on the PSA, by a method wherein an antibody is acted on the PSA, by a method with a high performance liquid chromatography or with a mass spectrometry, or by an analysis method wherein these analysis methods are combined.

"PSA" used in the present invention may be a sample itself derived from a test subject, or the one extracted or purified therefrom. Alternatively, "PSA" may be a complex with antichymotrypsin and the like, or a free PSA, or a glycan or a glycopeptide prepared therefrom.

The present invention relates to a method using a high performance liquid chromatography (this may be abbreviated as HPLC) as an analysis means of PSA. For example, separation is made based on molecular weight of a detection object by a normal phase HPLC represented by an Amide column and the like, or based on orientation of a constituting glycan of a detection object by a reversed phase HPLC represented by an ODS column and the like. A pair of peaks corresponding to the LacdiNAc(+) glycan and the LacdiNAc(-) glycan are selected in the chromatogram obtained by analysis; and then the determining is made based on the R value calculated from heights or areas of the peaks. In detection, an arbitrary absorption wavelength or a fluorescent wavelength specific to a selected labeled compound may be used.

Alternatively, the present invention relates to a method using an analysis method involving acting a specifically bonding molecule to PSA and a combination thereof. The specifically bonding molecule includes several kinds of lectins,

an antibody, and a fragment thereof. For example, a specifically bonding molecule is reacted with a detection object that is immobilized on an ELISA plate, a polyvinylidene fluoride (PVDF) membrane, a glass- or a silicon-made array chip, or the like, and then, the LacdiNAc structure and the entire detection object are quantitatively analyzed.

For detection, by using a lectin or an antibody, that is bonded to an enzyme such as alkaline phosphatase and horseradish peroxidase, chemical color production using bromochloroindolyl phosphoric acid (BCIP) or nitroblue tetrazolium (NBT) via an enzymatic reaction, or chemical luminescence using a luminol reagent, or surface plasmon resonance may be used. In a related matter, the detection object is bonded to an immobilized specifically bonding molecule; and then a series of detection may be carried out by reacting the bonded object with a different kind of a specific lectin or a specific antibody. In the present invention, "specific antibody" may be abbreviated as merely "antibody" and "specific lectin" as merely "lectin".

In the case that a lectin is used as the specifically bonding molecule, the LacdiNAc(+) glycan may be detected by acting, for example, a WFA (wisteria floribunda agglutinin) lectin, capable of recognizing a non-reduced GalNAc terminal, to PSA. In the case that an antibody is used as the specifically bonding molecule, commercially available polyclonal antibody or monoclonal antibody may be used as the antibody to PSA.

In relation to a method wherein an antibody capable of recognizing a specific glycan structure is used as the specifically bonding molecule, the present invention relates to the following step to prepare a polyclonal antibody and a monoclonal antibody capable of recognizing the LacdiNAc structure. Firstly, a glycan structure containing the LacdiNAc structure, its analog, or its derivative is prepared from a biological sample or synthesized chemically or biochemically to obtain a bonded polyvalent body with the structure. Then, an animal is immunized with the bonded polyvalent body together with an immunoreaction-activating substance. In this instance, the bonded polyvalent body is preferably bonded to the immunoreaction-activating substance, wherein the bonded body is used for immune singly or together with an immunoreaction-activating substance further. More preferably, the bonded polyvalent body is given, together with at least one kind of adjuvant molecules, to an antibody-producing organism by a syringe or by mucosal delivery.

## Examples

Hereinbelow, the present invention will be explained more specifically by showing Examples; but the present invention is not limited to them unless beyond its scope.

## Example 1

### (a) Step (1): Isolation of PSA

Firstly, immunoglobulin in serum was removed. Four milliliters of protein A agarose (manufactured by PIERCE Inc.) was packed into a disposable plastic column (manufactured by PIERCE Inc.) and then equilibrated with a phosphate-buffered saline(PBS). A mixture of PBS and serum of a test subject diagnosed with BHP (T-16) was charged into the column packed with protein A agarose; and then the column was rinsed with 2-fold column volume (CV) of PBS. A fraction containing PSA not bonded to the carrier was collected and added with $Na_2SO_4$ such that its final concentration would be 1M.

Then, albumin, which is a main protein in serum, was removed. One milliliter of Fractogel (registered trade name) EMD TA(S) (manufactured by Merck & Co., Inc.) was packed into a disposable plastic column and then equilibrated with 20 mM phosphate buffer solution (pH 7.4, containing 1M of $Na_2SO_4$). The foregoing fraction containing PSA was charged into a column packed with Fractogel; and then the column was rinsed with 7 CV of the buffer solution by using a charging pump. Then, adsorbed protein was eluted with 20 mM phosphate buffer solution (pH 7.4, not containing $Na_2SO_4$) to obtain a PSA-containing fraction.

Then, PSA was liberated from a complex of serum PSA with $a_1$-antichymotrypsin (PSA-ACT). The same amount of 4 M ethanolamine solution (pH 10.5) was added to the obtained PSA-containing fraction, so that the final ethanolamine concentration would be 2 M; and the resulting solution was shaken at 25°C for 14 hours to conduct a reaction. Thereafter, the reaction mixture was neutralized by adding 2 M hydrochloric acid with ice-cooling.

Then, an immunoprecipitation was carried out by using a PSA antibody. Firstly, a commercially purchased antihuman PSA-rabbit polyclonal antibody (manufactured by DAKO A/S) was bonded to Dynabeads (registered trade name) Protein G (manufactured by Invitrogen Corp.) and then treated with dimethyl pimelimidate (DMP, manufactured by PIERCE Inc.) to obtain antibody magnetic beads by crosslinking between the antibody and the magnetic beads. The obtained PSA antibody magnetic beads were added to the forgoing neutralized reaction mixture; and the resulting mixture was shaken at 4°C for one hour. Subsequently, the PSA antibody magnetic beads were rinsed with PBS containing 0.02% Tween-20 for three times and with PBS once. Then, 1 M propionic acid was added to the PSA antibody magnetic beads; and then the resulting mixture was shaken at 4°C for 40 minutes to elute a protein that was adsorbed to the PSA antibody magnetic beads for recovery. The eluted protein was dried by a centrifugal concentrator (SpeedVac).

The dried protein was analyzed by an enzyme-linked immunosorbent assay (ELISA, manufactured by Eiken Chemical Co., Ltd.) and an electrophoresis; as a result, it was found that approximately 50% of PSA contained originally in the serum could be recovered.

To 0.125 M Tris-HCl buffer solution (PH 6.8) were added 10% by mass of mercaptoethanol, 4% by mass of SDS, 10% by mass of sucrose, and 0.004% by mass of bromophenol blue to obtain a sample buffer. To 20 μL of the sample buffer was added PSA that was purified in step (1); and the resulting mixture was heated at 100°C for 3 minutes and then allowed to stand in an ice bath for cooling. The obtained sample was separated by electrophoresis on 10% by mass of polyacrylamide gel. After separation by the electrophoresis, the polyacrylamide gel was lightly rinsed with purified water and then dyed with SYPRO (registered trade name) Ruby (manufactured by Invitrogen Corp.). A gel portion containing the dyed protein was cut out and transferred to a 1.5-mL tube. The cut-out gel was rinsed sequentially with purified water, with 50% aqueous acetonitrile, and with acetonitrile; and then the gel was dried.

Separately, the foregoing procedures were followed by using 1 mL of serum having PSA concentration of 4 to 10 ng/mL; as a result, a strong PSA band was observed in a Western blot. From this result, it was demonstrated that PSA could be purified efficiently by the foregoing method in spite of such a low PSA concentration.

(b) Step (2): Preparation of glycan (PSA derivative)

Into the tube containing dry gel obtained in step (1) were added 10 mM dithiothreitol (DTT) and 25 mM aqueous ammonium bicarbonate (pH 8.0), and the resulting mixture was shaken for a reduction reaction at 56°C under the light-shield for one hour; and then the solution in the tube was removed. Into the tube was added 55 mM aqueous iodeacetamide to conduct an alkylation reaction by shaking the resulting mixture at room temperature under the light-shield for 45 minutes; and then the solution in the tube was removed. The gel after the reduction and the alkylation was rinsed with 25 mM aqueous ammonium bicarbonate (pH 8.0) and with acetonitrile; and then the gel was dried.

Into the gel thus obtained were added a solution containing 250 ng of Lysyl Endopeptidease (registered trade name (mass spectrometry grade), manufactured by Wako Pure Chemical Industries, Ltd.) and 25 mM aqueous ammonium bicarbonate (pH 8.0); and then the resulting mixture was allowed to stand in an ice bath for 45 minutes to swell the gel. Thereafter, the reaction mixture was gently stirred at 37°C for 18 hours. After 75% aqueous acetonitrile (containing 0.1% of trifluoroacetic acid) was added into the mixture, extraction was carried out by shaking the resulting mixture for 20 minutes to recover a solution.

The solution thus recovered was dried by a centrifugal concentrator. Into the resulting residue were added 50 mM aqueous ammonium bicarbonate (pH 8.0) containing 1 μg of Pefablock SC (manufactured by Roche Diagnostics K. K.) and thereafter 1 unit (1 unit/μL) of peptide N-glycosidase F (manufactured by Sigma Corporation); and then the resulting mixture was shaken at 37°C for 18 hours. After completion of shaking, 1 μL of 1% aqueous trifluoroacetic acid was added to the reaction mixture. Suction and discharge of the reaction mixture by using C18 chips packed with an octadecyl (C18) silica carrier were repeated to adsorb a peptide thereby separating it from a glycan in the reaction mixture to obtain a glycan fraction. Subsequently, the peptide was eluted from the C18 chips by using 70% aqueous acetonitrile containing 0.1% trifluoroacetic acid to obtain a peptide fraction. From the peptide fraction thus obtained, a peptide derived from PSA was detected.

Into a microspin column was packed 30 mg of carbon graphite (manufactured by GL Sciences Inc.), which was then rinsed. Into the graphite-packed microspin column was added the glycan fraction; and then 5% aqueous acetonitrile (0.1% trifluoroacetic acid) was added as a rinsing liquid to carried out rinsing by centrifugal separation (300xg for one to two minutes). After the rinsing liquid was removed, 50% aqueous acetonitrile (0.1% trifluoroacetic acid) was added; and then a glycan was eluted by centrifugal separation (300xg for one to two minutes) to obtain an eluted glycan solution. The eluted glycan solution thus recovered was dried by using a centrifugal concentrator, and then redissolved into 2 μL of pure water to obtain a glycan solution.

(c) Step (3): Labeling of glycan (PSA derivative)

Onto a target plate for MALDI was dropped 0.5 μL of the glycan solution obtained in step (2), and then it was dried by air. Then, onto the target plate was dropped 0.25 μL of DMSO solution of 1-pyrenyl diazomethane (PDAM, 500 pmol), and then it was dried at 40°C for about 25 minutes. With this, a glycan labeled with PDAM was obtained on the target plate.

(d) Step (4): MS analysis

On to the target plate to which the labeled glycan obtained in step (3) was supported was dropped 0.5 μL of 50% acetonitrile solution containing 2,5-dihydroxy benzoic acid (DHBA, concentration of 10 mg/mL), and then it was dried at room temperature.

The target plate thus obtained was set in a MALDI-QIT-TOF MS instrument (AXIMA-QIT; manufactured by Shimadzu/Kratos Analytical Corporation), and then MS analysis was carried out. To quantitatively compare, an area surrounded by peripheral that was larger than spread of the sample on the target plate was raster-scanned to accumulate all the significant signals. Typical MS spectrum obtained is shown in FIG. 1.

Separately, $MS^2$ analysis was carried out by using ions obtained at m/z=2077 and m/z=2118 as precursor ions. As a result, it was found that the signal P1 at m/z=2077 in FIG. 1 corresponds to the LacdiNAc(-)glycan shown by A in Chemical Formula 1, and the signal P2 at m/z=2118 corresponds to the LacdiNAc(+)glycan shown by B in Chemical Formula 1. Here, in the Formula, [Sia] designates N-acetylneuraminic acid, [Glc] designates glucose, [Gal] designates galactose, [Man] designates mannose, and [Fuc] designates fucose. Further, in the Formula, the brackets show a bond to either one of the two unreduced terminals. The same is applied to other chemical formulae shown hereinafter.

Result of the $MS^2$ analysis by using the signal at m/z=2118 as the precursor ion is shown in FIG. 2. Not only the fragment at m/z=1751 (ion $b_6$) showing existence of Sia-[Gal-GlcNAc-Man-(GalNAc-GlcNAc-Man-)Man-GlcNAc], the fragment at m/z=858 (ion $b_4$) corresponding to the structure of Sia-GalNAc-GlcNAc-Man, and so on, but also the fragment at m/z=696 (ion $b_3$) corresponding to the structure of Sia-GalNAc-GlcNAc including the LacdiNAc structure were confirmed. As a result, the precursor ion structure was confirmed to be Sia-[Gal-GlcNAc-Man-(GalNAc-GlcNAc-Man)Man-GlcNAc-(Fuc-)GlcNAc].

S(P1), the signal strength of P1, and S(P2), the signal strength of P2, were obtained; and then the ratio R Value=[S(P2)/S(P1)]×100 was calculated to be 12 (%).

[Chemical Formula 1]

A) [P1I] Sia (*m/z* =2077)

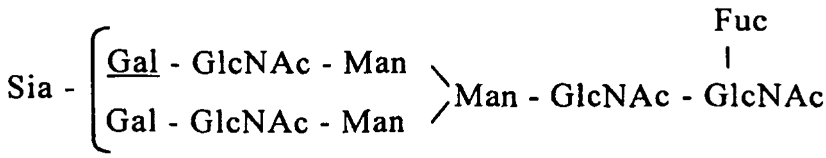

B) [P2I] (*m/z* =2118)

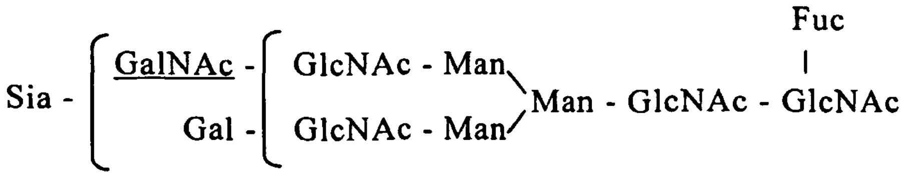

Examples 2 to 4

The same procedures as Example 1 were followed by using serums of two anonymous test subjects diagnosed as BHP, tagged with respective identification codes (Examples 1 and 2), and by using serums of two anonymous test subjects diagnosed as PC by biopsy, tagged with respective identification codes (Examples 3 and 4). Here, in time of carrying out the procedures, the informed consent was obtained from the test subjects after approval of ethics examination by a medical organization.

PSA concentration in the serum and percentage R value of signal strength ratio of LacdiNAc(-) (m/z=2077) and LacdiNAc(+) (m/z=2188) of each test subject are shown in the following Table 1.

[Table 1]

Table 1: Evaluation Results of Examples 1 to 4

| No. | Identificat ion Code | Disease Name | PSA Concentration in serum (ng/mL) | R-Value |
|---|---|---|---|---|
| Example 1 | T-16 | BPH | 40.4 | 12.0 |
| Example 2 | T-13 | BPH | 112.6 | 18.4 |
| Example 3 | T-17 | PC | 1450.0 | 65.0 |
| Example 4 | N-18 | PC | 769.3 | 95.0 |

MS spectrum obtained in Example 4 is shown in FIG. 3 as a typical example of the case that serum of a test subject that was diagnosed as PC is used. From the $MS^2$ analysis by the same procedures as Example 1, existence of glycans having the structures shown by C and D in Chemical Formula 2, in addition to the structures shown by A and B in Chemical Formula 1 was confirmed. "Pyrene" shown in the Formula means the pyrene label (1-pyrenylmethyl group) by PDAM. Further, in the Formula, the brackets show a bond to either one of two N-acetylneuraminic acids.
[Chemical Formula 2]

C) [P1II] (*m/z* =2582)

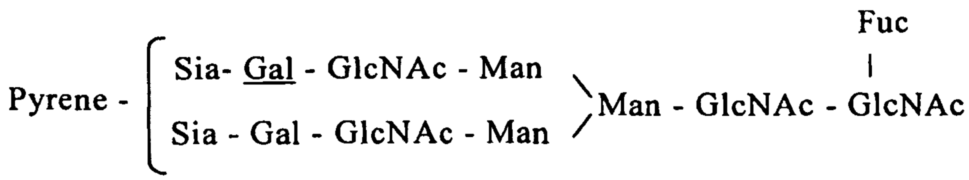

D) [P2II] (*m/z* =2623)

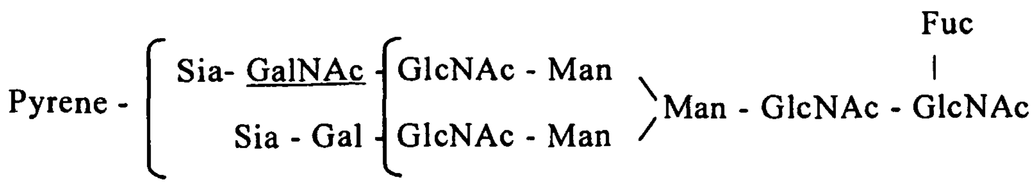

[Evaluation]

As shown in Table 1, percentage R values of signal strength ratio of LacdiNAc(+)/LacdiNAc(-) of the samples derived from serum of the test subjects diagnosed as PC were more than 30% ($R_{(s)}$), showing that a large amount of the GalNAc-substituted glycan is contained. On the other hand, percentage R values of signal strength ratio of the samples derived from serum of the test subjects diagnosed as BPH were less than 30% ($R_{(s)}$). Namely, it became clear for the first time that a larger amount of the LacdiNAc structure exists in the serum PSA glycan of the test subject diagnosed as PC as compared with the serum PSA of the test subject diagnosed as BPH.
In addition, it was found that the R value, the signal strength ratio LacdiNAc(+)/LacdiNAc(-), is not related to the serum PSA concentration in PC. This result shows that the method of the present invention may be used to distinguish between PC and BPH, independent of the PSA concentration. Accordingly, the method of the present invention can be applied to the sample of wide PSA concentration in serum. In particular, there is a possibility in the method of the present invention that PC and BPH can be effectively distinguished with high accuracy in the PSA concentration range in serum, namely, in the so-called gray zone (4 to 10 ng/mL).

Example 5

This Example shows that a glycopeptide can be used in place of a glycan of the foregoing Examples.
Without conducting PSA separation described in step (1) and reduction by DTT and alkylation by iodoacetamide

described in step (2) in Example 1, into 100 ng of standard PSA-ACT (manufactured by CORTEX BIOCHEM, Inc.) was added 50 mM aqueous ammonium bicarbonate (pH 8.0) containing 50 units of thermolysin (manufactured by Calbio Chem, Inc.); and then the resulting mixture was allowed to stand at 56°C for 18 hours for reaction. The reaction mixture was dried by a centrifugal concentrator. The obtained solid substance was dissolved into 0.8% aqueous trifluoroacetic acid; and then the resulting mixture was allowed to stand at 80°C for 40 minutes to carry out the elimination reaction of sialic acid. The reaction sample was dried by a centrifugal concentrator.

Subsequently, by using 50 mg of Intersep GC, which is a carbon graphite-packed cartridge (manufactured by GL Sciences Inc.), a glycopeptide was roughly purified and desalinated from the obtained sample. Firstly, into the cartridge were sequentially added 1M aqueous NaOH, distilled water, 30% aqueous acetic acid, and "aqueous solution containing 5% acetonitrile and 1% trifluoroacetic acid" to rinse and equilibrate the carbon graphite. Then, the dried glycopeptide sample was dissolved in "aqueous solution containing 5% acetonitrile and 1% trifluoroacetic acid", and then the resulting mixture was sent to the cartridge. The glycopeptide adsorbed to the cartridge was rinsed by sequentially sending distilled water and "aqueous solution containing 5% acetonitrile and 1% trifluoroacetic acid". Thereafter, "aqueous solution containing 80% acetonitrile and 1% trifluoroacetic acid" was sent to the cartridge to elute the adsorbed glycopeptide. The obtained glycopeptide fraction was recovered and dried by a centrifugal concentrator.

The glycopeptide was purified by using Sepharose CL4B (manufactured by Sigma-Aldrich Co.). Prior to use, Sepharose was rinsed with 50% aqueous ethanol for five times, and then with a mixture solution of "butanol:distilled water:ethanol=4:1:1" for five times. The roughly purified dry glycopeptide was dissolved into the mixture solution, to which was then added 6 μL of the rinsed Sepharose; and then the resulting mixture was shaken at room temperature for one hour to adsorb the glycopeptide to Sepharose. Then, the Sepharose was rinsed for nine times by using the foregoing mixture solution. To the Sepharose adsorbed with the glycopeptide was added 50% aqueous ethanol; and then the resulting mixture was shaken at room temperature for 30 minutes to elute the glycopeptide. A solution containing the eluted glycopeptide was recovered and then dried by a centrifugal concentrator. Then, the dried glycopeptide was dissolved into 4 μL of 5% aqueous acetonitrile.

The glycopeptide solution thus obtained was subjected to step (3) and step (4) of Example 1, and then the MS analysis was carried out. The MS spectrum thus obtained is shown in FIG. 4. Spectrum of the PSA-derived 43-47 peptide attached with a glycan was obtained; and two kinds of the glycopeptides, P1 and P2, corresponding to GalNAc/Gal substitution with mass difference of 41, could be detected. The respective compositions are shown by E and G (P1) and by F and H (P2) in Chemical Formula 3. Here, "Peptide" in the Formula shows a peptide fragment derived from PSA.

[Chemical Formula 3]

E) [$P1_a$] ($m/z$ =2386)

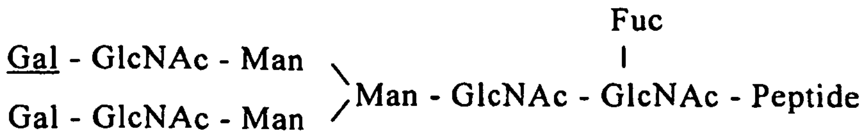

F) [$P2_a$] ($m/z$ =2427)

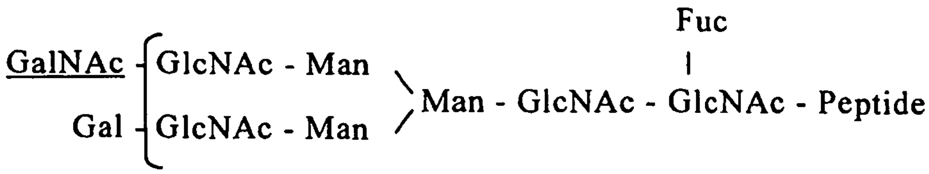

G) [$P1_b$] ($m/z$ =2240)

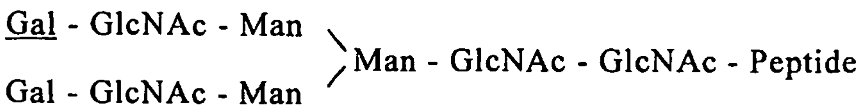

H) [$P2_b$] ($m/z$ =2281)

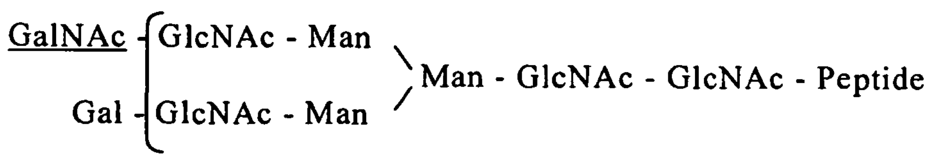

An average of percentage R value of signal strength ratio of LacdiNAc(+)/LacdiNAc(-) of the standard PSA-ACT derived from a seminal plasma was 25%.

As can be seen above, percentage R value of signal strength ratio of LacdiNAc(+)/LacdiNAc(-) of the standard PSA was less than 30%, which was close to the result of glycan analysis of the test subject suffered from BPH and different from the R value in the glycan analysis of the test subject suffered from PC. Further, when the glycopeptide is used, because of existence of the glycopeptide bond, identification of PSA and confirmation of the site of glycan attachment become possible. With this, percentage R value of signal strength ratio of LacdiNAc(+)/LacdiNAc(-) can be obtained accurately even if other glycoproteins are present as a mixture.

Example 6

The glycopeptide analysis was carried out for a serum of the test subject (N-18) suffered from PC shown in Example 4. By using the serum of the test subject, step (1), and reduction by DTT, alkylation by iodoacetamide, rinsing, and drying as described in step (2) in Example 1 were carried out to obtain dried gel.

Then, into the dried gel was added 25 mM aqueous ammonium bicarbonate (pH 8.0) containing 1 to 100 units of thermolysin (manufactured by Calbio Chem, Inc.); and then the resulting mixture was allowed to stand in an ice bath for 45 minutes to swell the gel. The swollen gel was lightly stirred at 56°C for 18 hours. To the gel thus obtained was added "aqueous solution containing 70 to 80% acetonitrile and 0.1% trifluoroacetic acid"; and then the resulting mixture was shaken for 20 minutes to recover a solution.

Into 25 mg of rinsed ZIC (registered trade name) HILIC solid phase column packing material (manufactured

by SeQuant AB) was added the solution thus recovered. Then, it was rinsed with "aqueous solution containing 80% acetonitrile and 0.1% trifluoroacetic acid"; and then a glycopeptide was eluted by using 0.1% aqueous trifluoroacetic acid. The eluted glycopeptide solution was dried by a centrifugal concentrator; and then the solid substance was dissolved into 2 μL of pure water to obtain a glycopeptide solution.

The glycopeptide solution thus obtained was subjected to the procedures similar to those in step (3) of Example 1 to label the glycopeptide. Then, the target plate thus obtained was subjected to the procedures similar to those in step (4) of Example 1 to carry out the MS analysis.

By the MS analysis, the glycopeptide having the PSA-derived 43-47 peptide attached with a glycan was detected. The obtained MS spectrum is shown in FIG. 5. As shown in FIG. 5, four pairs of the LacdiNAc(-)glycopeptide (P1) and the LacdiNAc(+)glycopeptide (P2), having mass difference of 41 corresponding to GalNAc/Gal substitution, were detected (Chemical Formula 4).

[Chemical Formula 4]

I) [P1b] (*m/z* =2021)

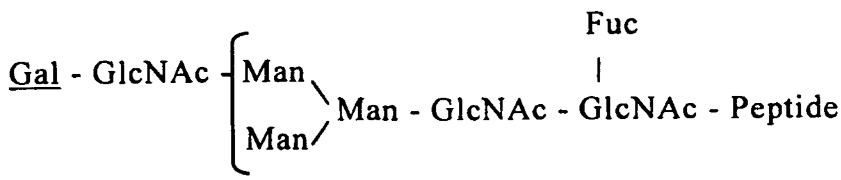

J) [P2b] (*m/z* =2062)

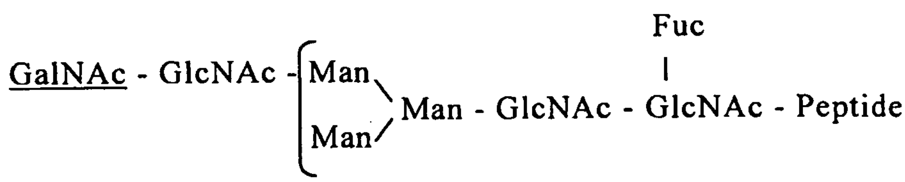

K) [P1d] (*m/z* =2600)

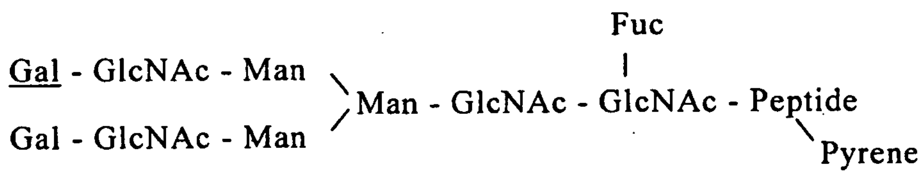

L) [P2d] (*m/z* =2641)

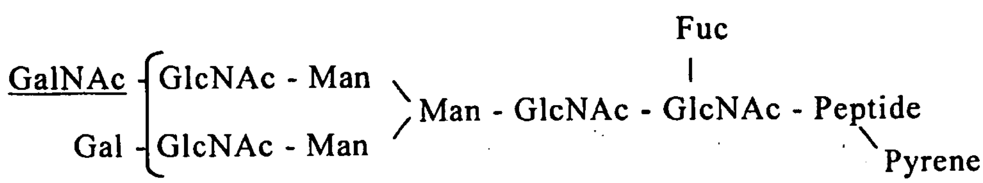

In any of the pairs, percentage R value of signal strength ratio of the LacdiNAc(+)/LacdiNAc(-)glycopeptides was more than 30%. Further, the R value obtained in this Example was similar to the R value of Example 4 in which the glycan analysis of the serum was carried out.

As mentioned above, even in the case that a glycopeptide was used as the PSA derivative, the signal pair of

glycopeptides having mass difference of 41 due to existence and non-existence of GalNAc/Gal substitution could be detected. In addition, percentage R value of signal pair strength ratio of the glycopeptides was 114±2%, showing the similar result, 95±3%, of percentage R value of signal pair strength ratio of the glycans. Accordingly, it is assumed that, in similar to the R value of the signal strength ratio of the LacdiNAc(+)/LacdiNAc(-) glycans shown in Examples 1 to 4, PC and BPH can be distinguished with high accuracy by measuring the R value of the signal strength ratio of the LacdiNAc (+)/LacdiNAc(-)glycopeptides.

Example 7

In Example 7, analysis of the patient's serum was carried out without conducting PSA isolation described in step (1) and reduction by DTT and alkylation by iodoacetamide described in step (2) in Example 1.

Firstly, immunoglobulin was removed from the serum of a patient suffered from PC (N-122, PSA concentration of 186 ng/mL). Three milliliters of protein A agarose carrier (manufactured by PIERCE Inc.) was equilibrated with the phosphate-buffered saline(PBS). This was packed into a disposable plastic column (manufactured by PIERCE Inc.), to which was added a mixture of the patient's serum and PBS; and then the resulting mixture was shaken at 4°C for 30 minutes. A fraction containing PSA not bound to the carrier was recovered, and further the protein A agarose carrier was rinsed with three-fold carrier volume (3 CV) of PBS to recover a fraction; and then into a mixture of both fractions was added $Na_2SO_4$ such that its final concentration be 1 M.

Then, albumin which is a major protein in serum was removed from the PSA-containing fraction. Into a disposable plastic column was packed 2.5 mL of Fractogel (trade name) EMD TA(S) (manufactured by Merck & Co., Inc.); and then it was equilibrated with 20 mM phosphate buffer (pH 7.4, containing 1 M of $Na_2SO_4$). Into the Fractogel-packed column was added the above-obtained fraction; and then 7 CV of the buffer solution was charged by a pump to remove albumin. Then, a protein adsorbed onto Fractogel (trade name) was eluted with 20 mM phosphate buffer (pH 7.4, not containing $Na_2SO_4$) to obtain a PSA-containing fraction. Subsequently, the fraction thus obtained was added to 2 mL of PBS-equilibrated protein A agarose carrier; and then the resulting mixture was shaken at 4°C for 30 minutes to remove unremoved immunoglobulin. A mixed solution of the fraction containing PSA not bound to the protein A agarose carrier and the fraction rinsed with 3 CV of PBS was used in the subsequent immunoprecipitation.

Subsequently, anti-PSA antibody beads to be used for immunoprecipitation were prepared. NHS-activated Sepharose 4 Fast Flow (manufactured by GE Healthcare Biosciences, Inc.) was rinsed with 1 mM HCL for three times. Then, into the washed beads was added a commercially purchased antihuman PSA-rabbit polyclonal antibody (manufactured by DAKO A/S); and then the resulting mixture was shaken at room temperature for 30 minutes to carry out crosslinking of the antibody with the beads. Subsequently, 0.5 M aqueous monoethanolamine containing 0.5 M of NaCl was added; and then the resulting mixture was shaken at room temperature for 30 minutes to inactivate remaining active groups. The anti-PSA antibody beads thus obtained were rinsed alternately with 0.1 M sodium acetate buffer (pH 4.0) containing 0.5 M NaCl and with 1 M Tris buffer (pH 9.0) containing 0.5 M NaCl for three times. Then, the anti-PSA antibody beads were rinsed with PBS for three times. Lastly, the beads were rinsed with PBS containing 0.02% of sodium azide, and then kept at 4°C until its use.

Then, immunoprecipitation was carried out. Into the foregoing mixed fraction solution containing PSA was added 0.1 mL of the anti-PSA antibody beads; and then the resulting mixture was shaken at 4°C for one hour. Subsequently, the anti-PSA antibody beads were transferred to Micro Bio-Spin chromatography column (manufactured by Bio-Rad Laboratories, Inc.). The anti-PSA antibody beads column was rinsed with PBS for four times, with PBS containing 0.02% of Tween-20 for three times, and with distilled water twice. Then, 1 M of aqueous propionic acid was added to the anti-PSA antibody beads column to recover a protein adsorbed onto the beads by elution. Elution by the aqueous propionic acid was repeated for ten times in total; and the eluted solutions were mixed and dried by a centrifugal concentrator.

A part of the obtained protein fraction was subjected to the Western blotting method for semi-quantitative analysis; then it was found that more than 70% of PSA contained in the original serum could be recovered.

Subsequently, according to Example 5, digestion by thermolysin, elimination reaction of sialic acid, rough purification by the carbon graphite-packed cartridge, and purification by using Sepharose CL4B were carried out sequentially to obtain a glycopeptide fraction. A glycopeptide of the glycopeptide solution thus obtained was labeled by conducting similar procedures to those of step (3) in Example 1. Then, the target plate thus obtained was subjected to the procedures of step (4) in Example 1; and then the MS analysis was carried out.

By the MS analysis, the glycopeptide having the PSA-derived 43-47 peptide attached with a glycan was detected. The obtained MS spectrum is shown in FIG. 6. By the MS analysis, the signal pair of glycopeptides having mass difference of 41 due to existence and nonexistence of the LacdiNAc structure, P1 and P2, could be detected.

Examples 8 to 12

The same procedures as Example 7 were followed by using serums of five test subjects diagnosed as PC by biopsy, tagged with respective identification codes (Examples 8 to 12). Here, in time of carrying out the procedures, the informed consent was obtained from the test subjects after approval in ethics examination by a medical organization.

PSA concentration in serum of each test subject including Examples 5, 6, and 7, and percentage R value of signal strength ratio of the LacdiNAc(-) glycopeptide (m/z=2386) and the LacdiNAc(+) glycopeptide (m/z=2427) are shown in the following Table 2.

[Table 2]

Table 2: Evaluation Results of Examples 5 to 12

| No. | Identification Code | Disease Name | PSA Concentration in serum (ng/mL) | R-Value |
|---|---|---|---|---|
| Example 5 | Standard PSA-ACT | - | - | 25.0 |
| Example 6 | N-18 | PC | 769.3 | 114.0 |
| Example 7 | N-122 | PC | 186.2 | 86.0 |
| Example 8 | T-15 | PC | 32.1 | 52.0 |
| Example 9 | T-33 | PC | 45.7 | 64.0 |
| Example 10 | N-86 | PC | 53.7 | 136.0 |
| Example 11 | T-23 | PC | 62.2 | 43.0 |
| Example 12 | N-99 | PC | 90.3 | 44.0 |

As can be seen in the above, the glycopeptide could be purified from the serum of a prostate carcinoma patient having low PSA concentration (32 ng/mL), thereby enabling to detect the glycopeptide by the MS analysis. It was also found that the R value of a prostate carcinoma patient having low serum PSA concentration was far more than 30%, in similar to the R value of a prostate carcinoma patient having large amount of PSA.

Accordingly, a glycopeptide could be directly prepared from a PSA-ACT complex in serum and from a free PSA without preparation of a free PSA from the complex in serum by the procedures as shown in Example 1, thereby enabling to detect the LacdiNAc(+) glycopeptide and the LacdiNAc(-) glycopeptide of PSA. This method can shorten the steps so that the method can become convenient and prompt, and in addition, recovery yield of the glycopeptide can be improved. Accordingly, the present method may be preferably applicable to a clinical sample.

## Claims

1. A method for determining prostate carcinoma, wherein
the method includes a step of analyzing a PSA glycan structure in a sample derived from a test subject, and
prostate carcinoma is determined in the case that amount of a glycan having LacdiNAc (N-acetylgalactosamine-N-acetylglucosamine) (LacdiNAc(+)) is more than 30% of amount of a glycan not having LacdiNAc but having LacNAc (galacotose-N-acetylglucosamine) (LacdiNAc(-)).

2. The method for determining prostate carcinoma according to claim 1, wherein
the method includes a step of analyzing a PSA glycan structure in a sample derived from a test subject,
prostate carcinoma is determined in the case that amount of a glycan having LacdiNAc (N-acetylgalactosamine-N-acetylglucosamine) (LacdiNAc(+)) is more than 30% of amount of a glycan not having LacdiNAc but having LacNAc (galacotose-N-acetylglucosamine) (LacdiNAc(-)), and
benign prostatic hyperplasia is determined in the case of 30% or less.

3. The method for determining prostate carcinoma according to claim 1, wherein analysis of the PSA glycan structure is done by a method wherein a lectin is acted on the PSA, by a method wherein an antibody is acted on the PSA,

by a method with a high performance liquid chromatography or with a mass spectrometry, or by an analysis means wherein these analysis methods are combined.

**4.** The method for determining prostate carcinoma according to claim 2, wherein analysis of the PSA glycan structure is done by a method wherein a lectin is acted on the PSA, by a method wherein an antibody is acted on the PSA, by a method with a high performance liquid chromatography or with a mass spectrometry, or by an analysis means wherein these analysis methods are combined.

**5.** The method for determining prostate carcinoma according to claim 3, wherein the method includes:

(1) a step of purifying PSA from a sample derived from a test subject,
(2) a step of preparing a PSA derivative from PSA purified in step (1),
(3) a step of labeling the PSA derivative obtained in step (2), and
(4) a step of analyzing the labeled PSA derivative obtained in step (3) by a mass spectrometry method; wherein a pair of signals shown by the mass difference of 41 due to presence and absence of a LacdiNAc (N-acetylgalactosamine-N-acetylglucosamine) structure is selected, and
prostate carcinoma is determined in the case that a signal strength derived from the glycan having LacdiNAc (LacdiNAc(+)) is more than 30% of a signal strength derived from the glycan not having LacdiNAc but having LacNAc (galacotose-N-acetylglucosamine) (LacdiNAc(-)).

**6.** The method for determining prostate carcinoma according to claim 4, wherein the method includes:

(1) a step of purifying PSA from a sample derived from a test subject,
(2) a step of preparing a PSA derivative from PSA purified in step (1),
(3) a step of labeling the PSA derivative obtained in step (2), and
(4) a step of analyzing the labeled PSA derivative obtained in step (3) by a mass spectrometry method; wherein

a pair of signals shown by the mass difference of 41 due to presence and absence of a LacdiNAc (N-acetylgalactosamine-N-acetylglucosamine) structure is selected,
prostate carcinoma is determined in the case that a signal strength derived from the glycan having LacdiNAc (LacdiNAc(+)) is more than 30% of a signal strength derived from the glycan not having LacdiNAc but having LacNAc (galacotose-N-acetylglucosamine) (LacdiNAc(-)), and
benign prostatic hyperplasia is determined in the case of 30% or less.

**7.** The method for determining prostate carcinoma according to claim 5, wherein the PSA derivative prepared in step (2) is a glycan derived from PSA or a glycopeptide derived from PSA.

**8.** The method for determining prostate carcinoma according to claim 6, wherein the PSA derivative prepared in step (2) is a glycan derived from PSA or a glycopeptide derived from PSA.

**Patentansprüche**

**1.** Verfahren zum Nachweis von Prostatakrebs, wobei
das Verfahren einen Schritt des Analysierens einer PSA-Glycan-Struktur in einer Probe, die von einem Probanden stammt, umfasst; und
Prostatakrebs nachgewiesen wird, wenn die Menge eines Glycans, das LacdiNAc (N-Acetylgalactosamin-N-acetylglucosamin) aufweist (LacdiNAc(+)), mehr als 30% der Menge eines Glycans beträgt, das kein LacdiNAc aufweist, aber LacNAc (Galactose-N-acetylglucosamin) aufweist (LacdiNAc(-)).

**2.** Verfahren zum Nachweis von Prostatakrebs gemäß Anspruch 1, wobei
das Verfahren einen Schritt des Analysierens einer PSA-Glycan-Struktur in einer Probe, die von einem Probanden stammt, umfasst;
Prostatakrebs nachgewiesen wird, wenn die Menge eines Glycans, das LacdiNAc (N-Acetylgalactosamin-N-acetylglucosamin) aufweist (LacdiNAc(+)), mehr als 30% der Menge eines Glycans beträgt, das kein LacdiNAc aufweist, aber LacNAc (Galactose-N-acetylglucosamin) aufweist (LacdiNAc(-)); und

eine benigne Prostatahyperplasie nachgewiesen wird, wenn die Menge 30% oder weniger beträgt.

3. Verfahren zum Nachweis von Prostatakrebs gemäß Anspruch 1, wobei die Analyse der PSA-Glycan-Struktur nach einem Verfahren, bei dem ein Lectin auf das PSA einwirken gelassen wird, nach einem Verfahren, bei dem ein Antikörper auf das PSA einwirken gelassen wird, nach einem Verfahren mit HPLC (high performance liquid chromatography) oder mit Massenspektrometrie oder nach einer Analysemethode, bei der diese Analyseverfahren miteinander kombiniert werden, erfolgt.

4. Verfahren zum Nachweis von Prostatakrebs gemäß Anspruch 2, wobei die Analyse der PSA-Glycan-Struktur nach einem Verfahren, bei dem ein Lectin auf das PSA einwirken gelassen wird, nach einem Verfahren, bei dem ein Antikörper auf das PSA einwirken gelassen wird, nach einem Verfahren mit HPLC (High performance liquid chromatography) oder mit Massenspektrometrie oder nach einer Analysemethode, bei der diese Analyseverfahren miteinander kombiniert werden, erfolgt.

5. Verfahren zum Nachweis von Prostatakrebs gemäß Anspruch 3, wobei
das Verfahren Folgendes umfasst:

(1) einen Schritt des Reinigens von PSA aus einer Probe, die von einem Probanden stammt;
(2) einen Schritt des Herstellens eines PSA-Derivats aus dem in Schritt (1) gereinigten PSA;
(3) einen Schritt des Markierens des in Schritt (2) erhaltenen PSA-Derivats; und
(4) einen Schritt des Analysierens des in Schritt (3) erhaltenen markierten PSA-Derivats durch ein Massenspektrometrieverfahren; wobei

ein Paar von Signalen, das sich durch die Massendifferenz von 41 aufgrund der Anwesenheit und Abwesenheit einer LacdiNAc-Struktur (N-Acetylgalactosamin-N-acetylglucosamin) bemerkbar macht, ausgewählt wird; und
Prostatakrebs nachgewiesen wird, wenn die Signalstärke, die auf das Glycan zurückgeht, das LacdiNAc aufweist (LacdiNAc(+)), mehr als 30% einer Signalstärke beträgt, die auf das Glycan zurückgeht, das kein LacdiNAc aufweist, aber LacNAc (Galactose-N-acetylglucosamin) aufweist (LacdiNAc(-)).

6. Verfahren zum Nachweis von Prostatakrebs gemäß Anspruch 4, wobei
das Verfahren Folgendes umfasst:

(1) einen Schritt des Reinigens von PSA aus einer Probe, die von einem Probanden stammt;
(2) einen Schritt des Herstellens eines PSA-Derivats aus dem in Schritt (1) gereinigten PSA;
(3) einen Schritt des Markierens des in Schritt (2) erhaltenen PSA-Derivats; und
(4) einen Schritt des Analysierens des in Schritt (3) erhaltenen markierten PSA-Derivats durch ein Massenspektrometrieverfahren; wobei

ein Paar von Signalen, das sich durch die Massendifferenz von 41 aufgrund der Anwesenheit und Abwesenheit einer LacdiNAc-Struktur (N-Acetylgalactosamin-N-acetylglucosamin) bemerkbar macht, ausgewählt wird; und
Prostatakrebs nachgewiesen wird, wenn die Signalstärke, die auf das Glycan zurückgeht, das LacdiNAc aufweist (LacdiNAc(+)), mehr als 30% einer Signalstärke beträgt, die auf das Glycan zurückgeht, das kein LacdiNAc aufweist, aber LacNAc (Galactose-N-acetylglucosamin) aufweist (LacdiNAc(-)); und
eine benigne Prostatahyperplasie nachgewiesen wird, wenn die Menge 30% oder weniger beträgt.

7. Verfahren zum Nachweis von Prostatakrebs gemäß Anspruch 5, wobei das in Schritt (2) hergestellte PSA-Derivat ein von PSA abgeleitetes Glycan oder ein von PSA abgeleitetes Glycopeptid ist.

8. Verfahren zum Nachweis von Prostatakrebs gemäß Anspruch 6, wobei das in Schritt (2) hergestellte PSA-Derivat ein von PSA abgeleitetes Glycan oder ein von PSA abgeleitetes Glycopeptid ist.

## Revendications

1. Procédé de détermination du carcinome de la prostate, dans lequel
le procédé comprend une étape consistant à analyser une structure glycanique du PSA dans un échantillon issu d'un sujet à tester, et
un carcinome de la prostate est déterminé dans le cas où la quantité d'un glycane contenant LacdiNAc (N-acétylgalactosamine-N-acétylglucosamine) (LacdiNAc(+)) est égale à plus de 30 % de la quantité d'un glycane ne contenant pas LacdiNAc mais contenant LacNAc (galactose-N-acétylglucosamine) (LacdiNAc(-)).

**2.** Procédé de détermination du carcinome de la prostate selon la revendication 1, dans lequel
le procédé comprend une étape consistant à analyser une structure glycanique du PSA dans un échantillon issu d'un sujet à tester,
un carcinome de la prostate est déterminé dans le cas où la quantité d'un glycane contenant LacdiNAc (N-acétylgalactosamine-N-acétylglucosamine) (LacdiNAc(+)) est égale à plus de 30 % de la quantité d'un glycane ne contenant pas LacdiNAc mais contenant LacNAc (galactose-N-acétylglucosamine) (LacdiNAc(-)), et
une hyperplasie bénigne de la prostate est déterminée dans le cas où elle est inférieure ou égale à 30 %.

**3.** Procédé de détermination du carcinome de la prostate selon la revendication 1, dans lequel l'analyse de la structure glycanique du PSA est réalisée au moyen d'un procédé dans lequel le PSA est soumis à l'action d'une lectine, au moyen d'un procédé dans lequel le PSA est soumis à l'action d'un anticorps, au moyen d'un procédé comprenant une chromatographie liquide haute performance ou une spectrométrie de masse, ou à l'aide d'un moyen d'analyse dans lequel ces procédés d'analyse sont combinés.

**4.** Procédé de détermination du carcinome de la prostate selon la revendication 2, dans lequel l'analyse de la structure glycanique du PSA est réalisée au moyen d'un procédé dans lequel le PSA est soumis à l'action d'une lectine, au moyen d'un procédé dans lequel le PSA est soumis à l'action d'un anticorps, au moyen d'un procédé comprenant une chromatographie liquide haute performance ou une spectrométrie de masse, ou à l'aide d'un moyen d'analyse dans lequel ces procédés d'analyse sont combinés.

**5.** Procédé de détermination du carcinome de la prostate selon la revendication 3, dans lequel
le procédé comprend :

(1) une étape consistant à purifier le PSA à partir d'un échantillon issu d'un sujet à tester,
(2) une étape consistant à préparer un dérivé de PSA à partir du PSA purifié à l'étape (1),
(3) une étape consistant à marquer le dérivé de PSA obtenu à l'étape (2), et
(4) une étape consistant à analyser le dérivé de PSA marqué obtenu à l'étape (3) au moyen d'un procédé de spectrométrie de masse ; dans laquelle
une paire de signaux identifiée par la différence de masse de 41, due à la présence et à l'absence d'une structure LacdiNAc (N-acétylgalactosamine-N-acétylglucosamine), est sélectionnée, et
un carcinome de la prostate est déterminé dans le cas où une intensité de signal issue du glycane contenant LacdiNAc (N-acétylgalactosamine-N-acétylglucosamine) (LacdiNAc(+)) est égale à plus de 30 % d'une intensité de signal issue du glycane ne contenant pas LacdiNAc mais contenant LacNAc (galactose-N-acétylglucosamine) (LacdiNAc(-)).

**6.** Procédé de détermination du carcinome de la prostate selon la revendication 4, dans lequel
le procédé comprend :

(1) une étape consistant à purifier le PSA à partir d'un échantillon issu d'un sujet à tester,
(2) une étape consistant à préparer un dérivé de PSA à partir du PSA purifié à l'étape (1),
(3) une étape consistant à marquer le dérivé de PSA obtenu à l'étape (2), et
(4) une étape consistant à analyser le dérivé de PSA marqué obtenu à l'étape (3) au moyen d'un procédé de spectrométrie de masse ; dans laquelle

une paire de signaux identifiée par la différence de masse de 41, due à la présence et à l'absence d'une structure LacdiNAc (N-acétylgalactosamine-N-acétylglucosamine), est sélectionnée,
un carcinome de la prostate est déterminé dans le cas où une intensité de signal issue du glycane contenant LacdiNAc (LacdiNAc(+)) est égale à plus de 30 % d'une intensité de signal issue du glycane ne contenant pas LacdiNAc mais contenant LacNAc (galactose-N-acétylglucosamine) (LacdiNAc(-)), et
une hyperplasie bénigne de la prostate est déterminée dans le cas où elle est inférieure ou égale à 30 %.

**7.** Procédé de détermination du carcinome de la prostate selon la revendication 5, dans lequel le dérivé de PSA préparé à l'étape (2) est un glycane dérivé du PSA ou un glycopeptide dérivé du PSA.

**8.** Procédé de détermination du carcinome de la prostate selon la revendication 6, dans lequel le dérivé de PSA préparé

à l'étape (2) est un glycane dérivé du PSA ou un glycopeptide dérivé du PSA.

fig.1

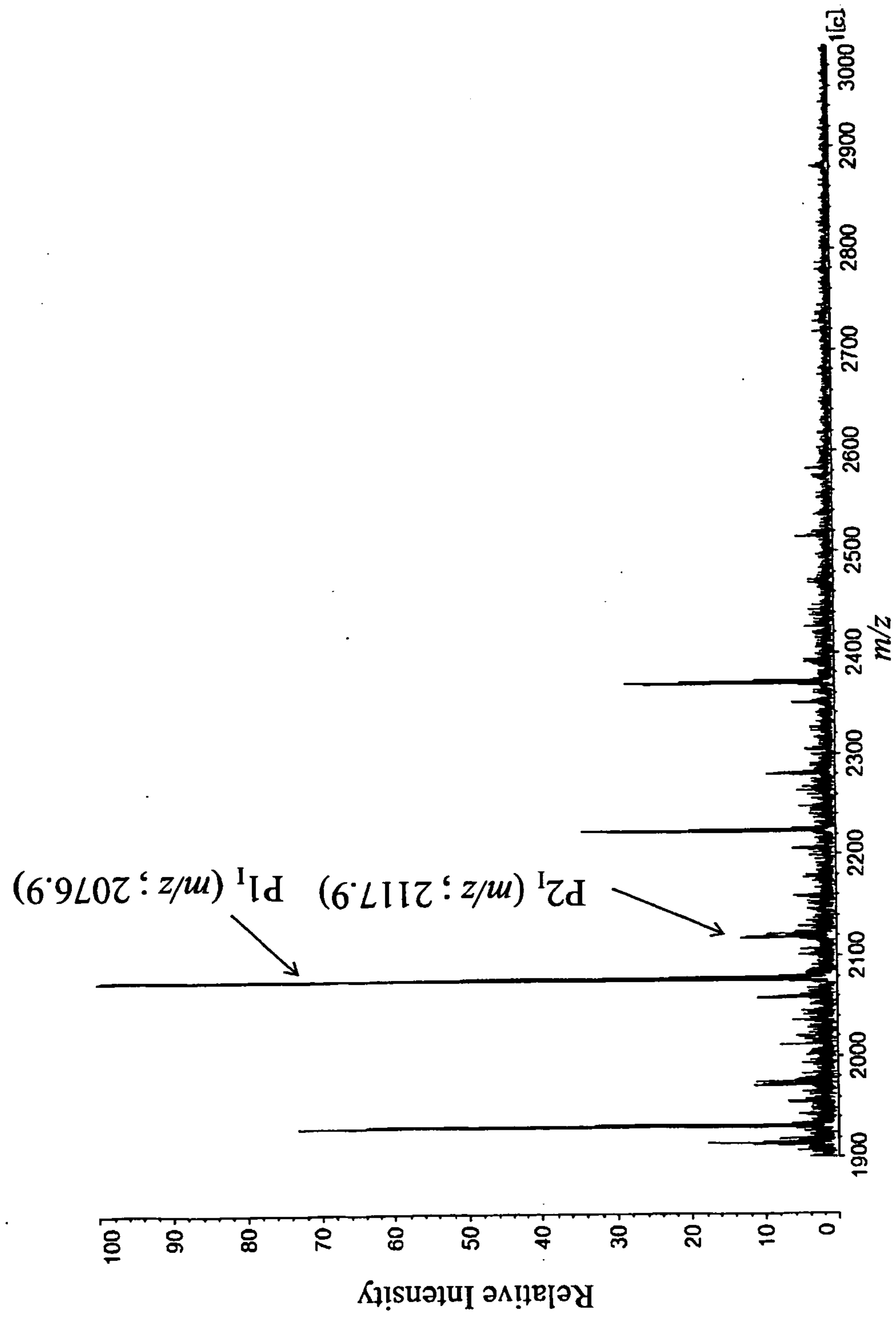

fig.2

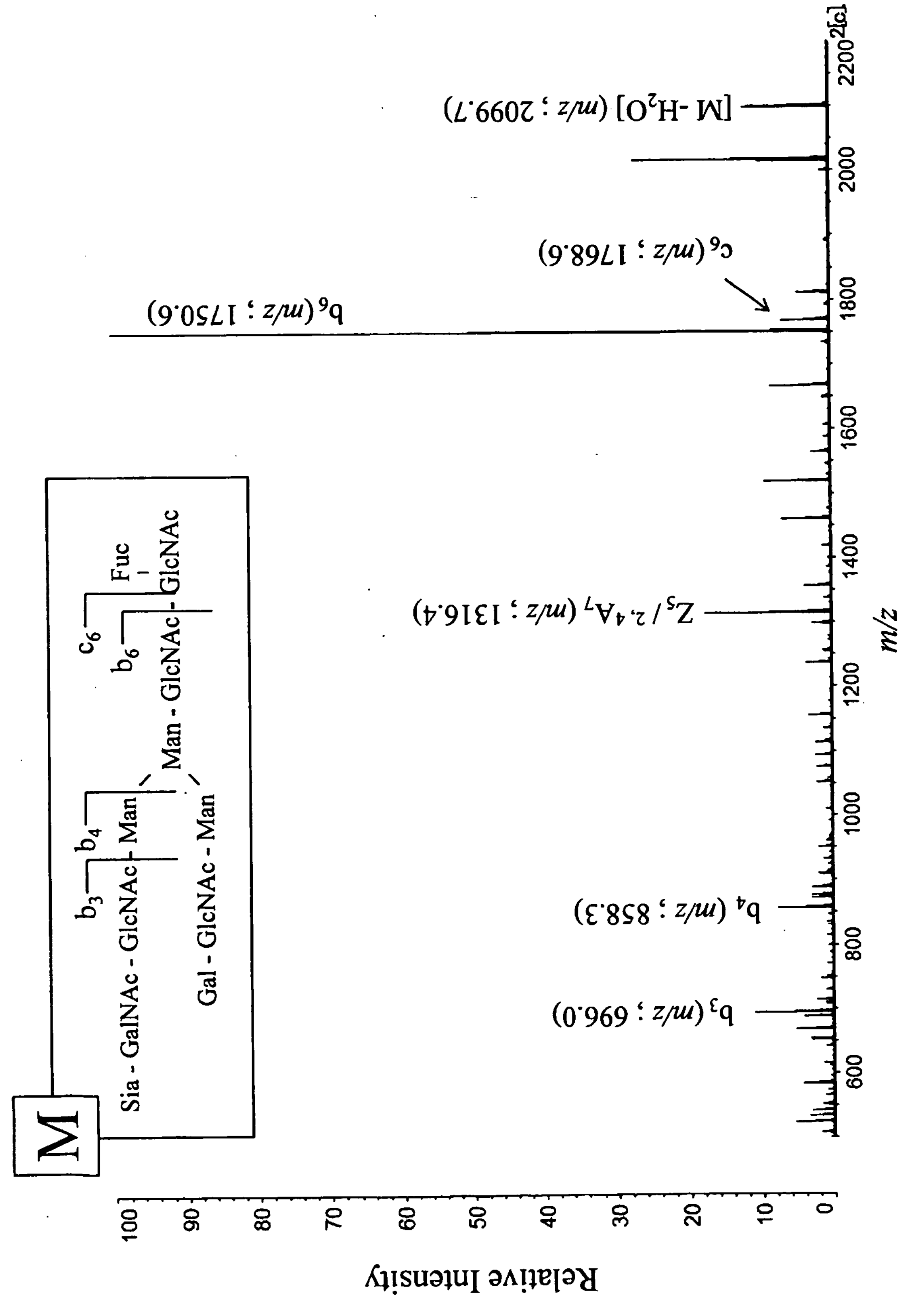
M
Sia - GalNAc - GlcNAc - Man
Gal - GlcNAc - Man
Man - GlcNAc - GlcNAc
Fuc
b3
b4
b6
c6
b3 (m/z ; 696.0)
b4 (m/z ; 858.3)
Z5 / 2,4A7 (m/z ; 1316.4)
b6 (m/z ; 1750.6)
c6 (m/z ; 1768.6)
[M -H2O] (m/z ; 2099.7)
Relative Intensity
m/z
0
10
20
30
40
50
60
70
80
90
100
600
800
1000
1200
1400
1600
1800
2000
2200

fig.3

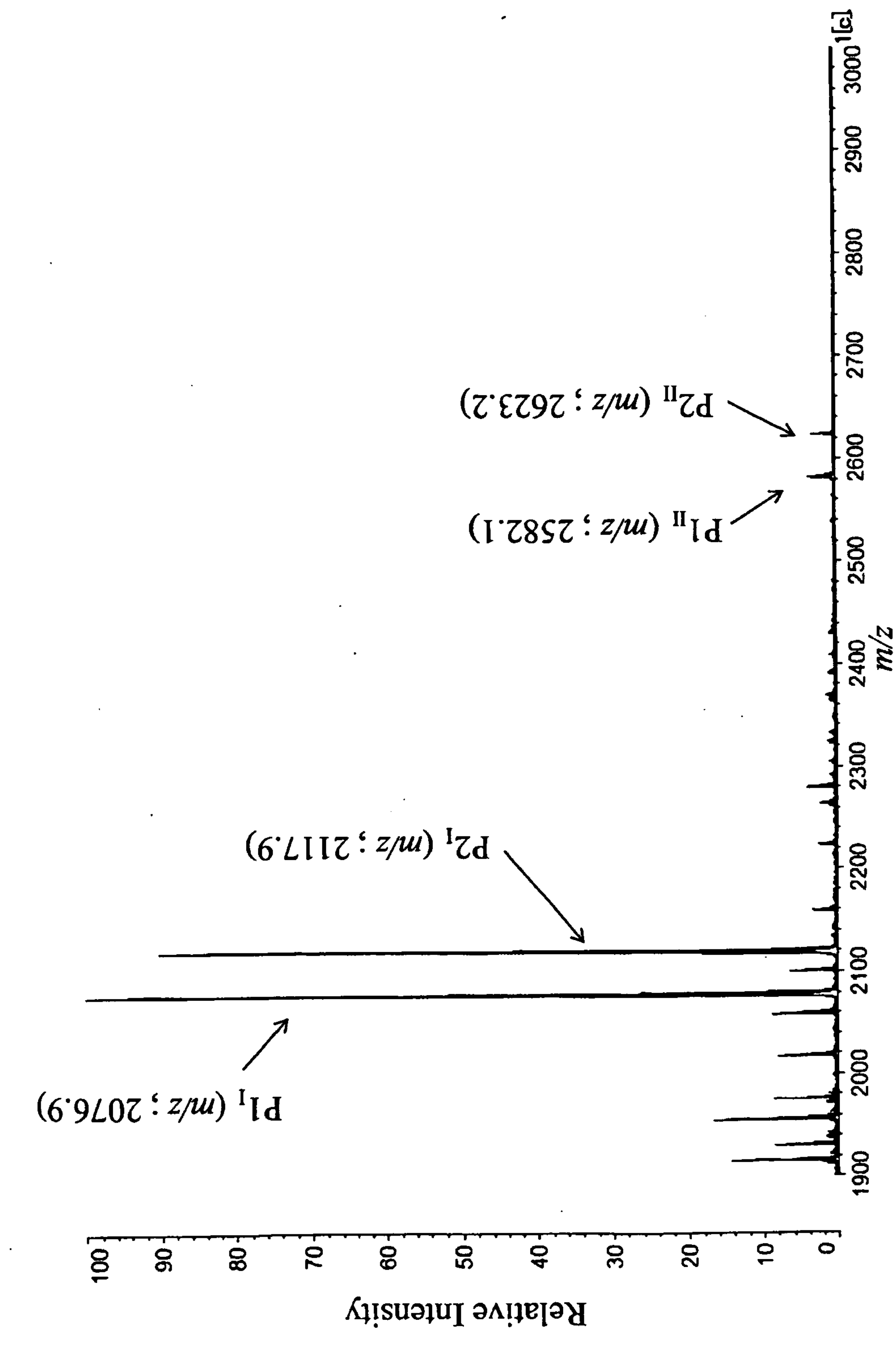
P1I (m/z ; 2076.9)
P2I (m/z ; 2117.9)
P1II (m/z ; 2582.1)
P2II (m/z ; 2623.2)
Relative Intensity
0 10 20 30 40 50 60 70 80 90 100
m/z
1900 2000 2100 2200 2300 2400 2500 2600 2700 2800 2900 3000
1[c]

fig.4

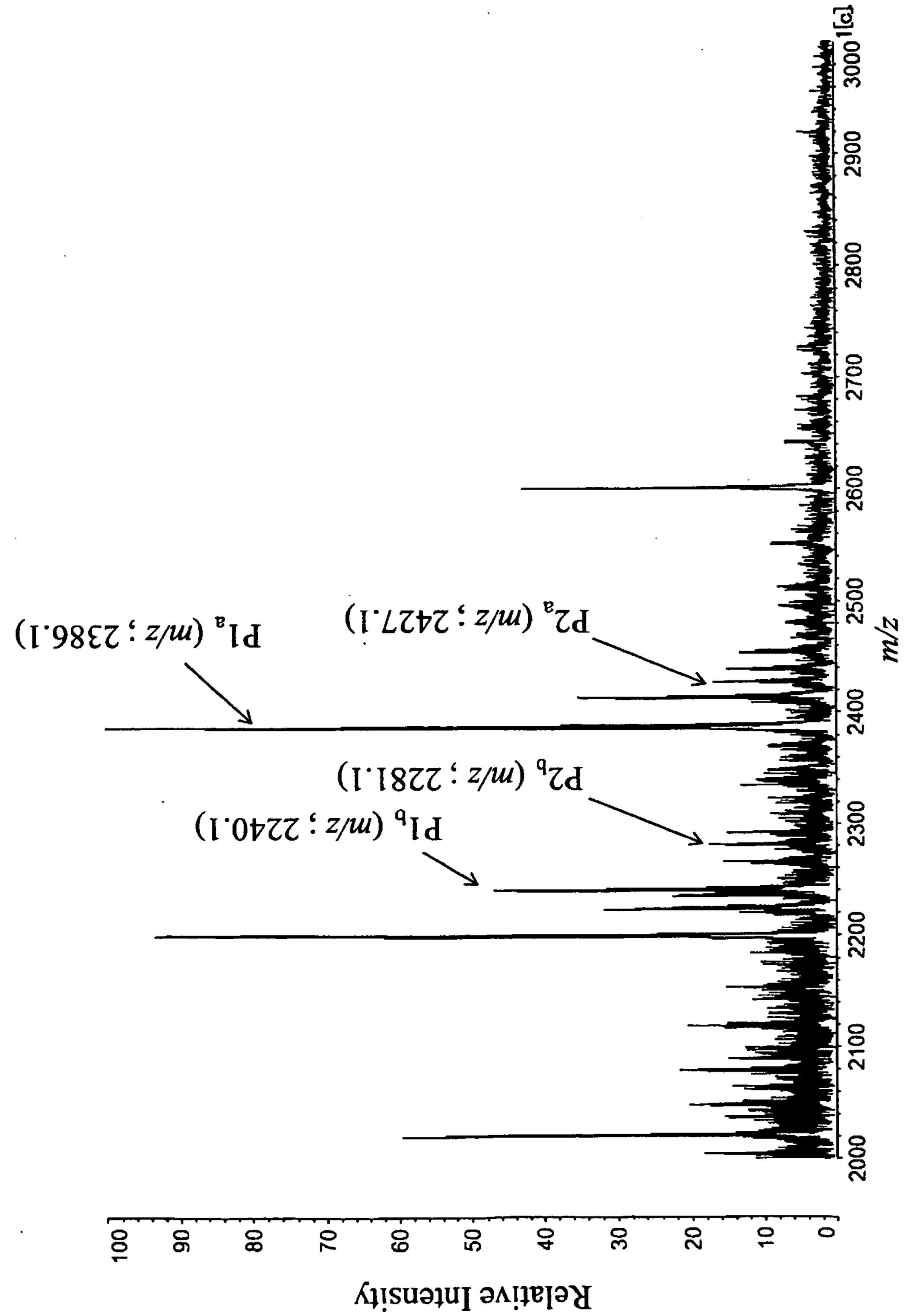
P1a (m/z ; 2386.1)
P2a (m/z ; 2427.1)
P2b (m/z ; 2281.1)
P1b (m/z ; 2240.1)
Relative Intensity
0
10
20
30
40
50
60
70
80
90
100
m/z
2000
2100
2200
2300
2400
2500
2600
2700
2800
2900
3000

fig.5

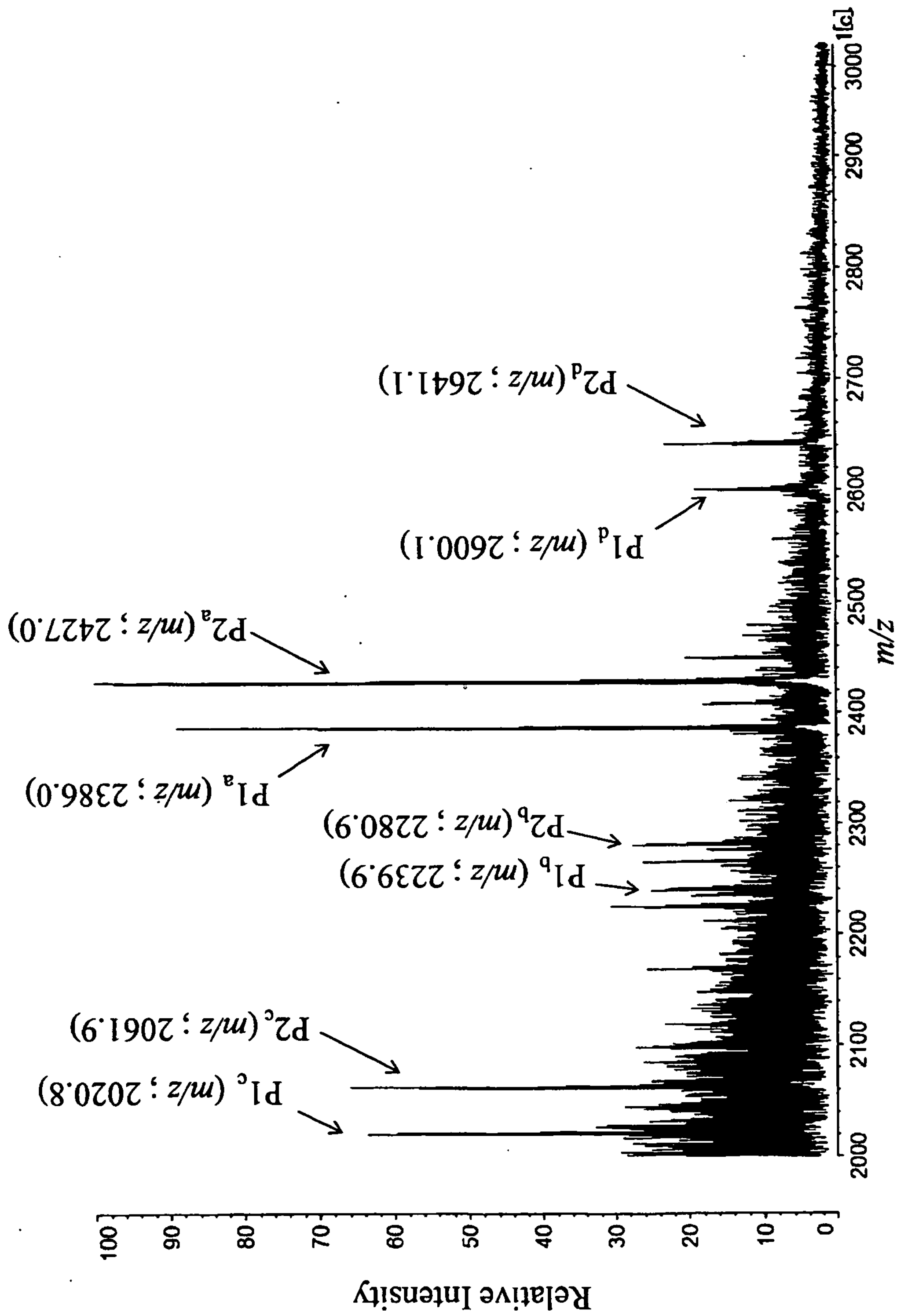
Relative Intensity
m/z
P1c (m/z ; 2020.8)
P2c (m/z ; 2061.9)
P1b (m/z ; 2239.9)
P2b (m/z ; 2280.9)
P1a (m/z ; 2386.0)
P2a (m/z ; 2427.0)
P1d (m/z ; 2600.1)
P2d (m/z ; 2641.1)

fig.6

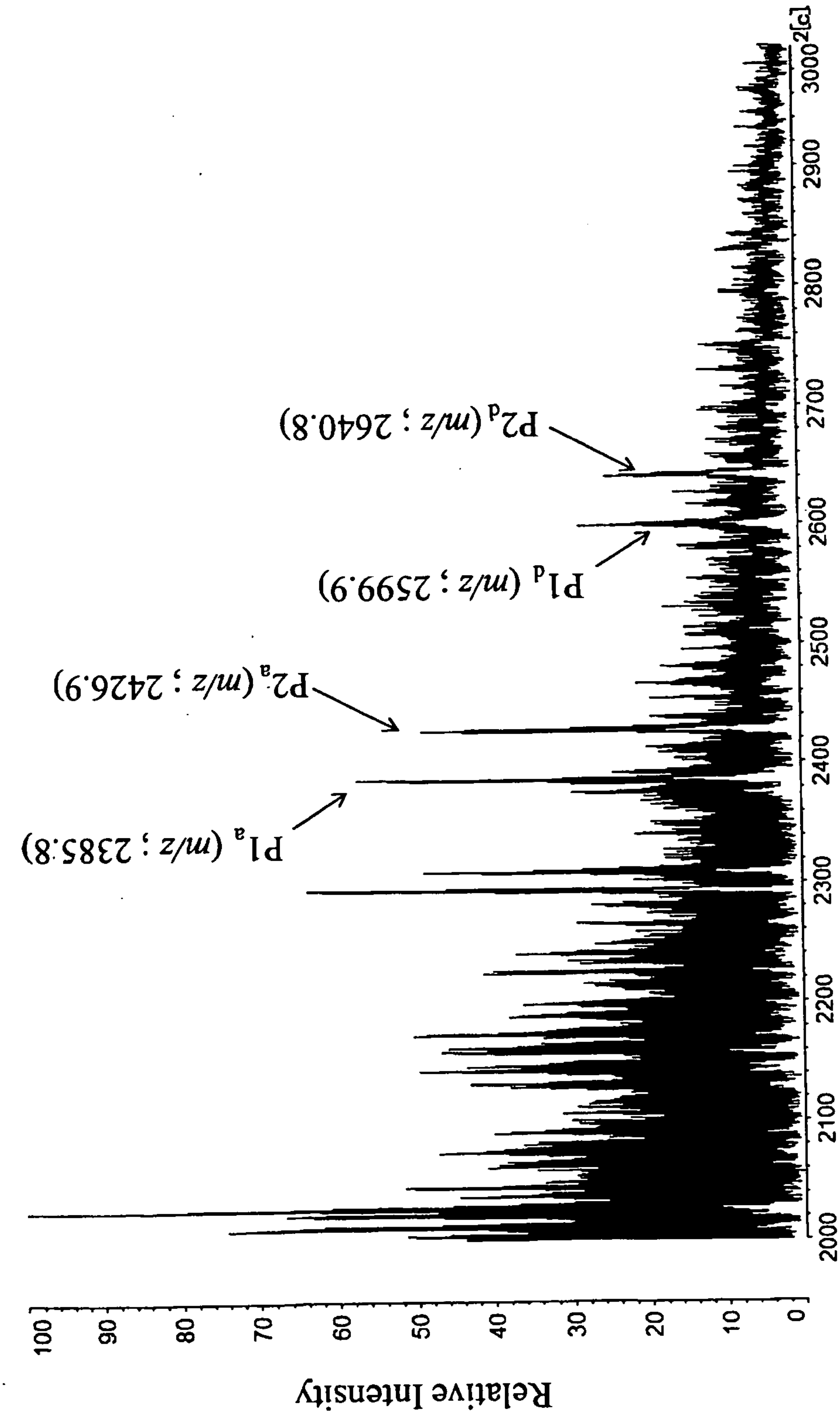
P1a (m/z ; 2385.8)
P2a (m/z ; 2426.9)
P1d (m/z ; 2599.9)
P2d (m/z ; 2640.8)
Relative Intensity
m/z
0
10
20
30
40
50
60
70
80
90
100
2000
2100
2200
2300
2400
2500
2600
2700
2800
2900
3000

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2002055108 A
- JP 2000514919 A
- JP 2005500057 A
- JP 2006515927 A


### Non-patent literature cited in the description


- **STAMEY et al.** *N. Engl. J. Med.,* 1987, vol. 317, 909-916
- **CATALONA et al.** *J. Am. Med. Assoc.,* 1998, vol. 279, 1542-1547
- **SUMI et al.** *J. Chromatogr. B,* 1994, vol. 727, 9-14
- **PERACAULA et al.** *Glycobiology,* 2003, vol. 13 (6), 457-470
- **PERACAULA et al.** *Glycobilology,* 2003, vol. 13 (4), 227-244
- **KUNO et al.** *Mol. Cell Proteomics,* 2009, vol. 8 (1), 99-108
- **TABARES et al.** *Glycobilology,* 2006, vol. 16 (2), 132-145
- **BINDUKUMAR et al.** *J. Chromatogr. B, Analyt. Technol. Biomed. Life Sci.,* 2004, vol. 813 (1-2), 113-120
- **ZHANG et al.** *Clin. Chem.,* 1995, vol. 41 (11), 1567-1573
- **OKADA et al.** *Biochim. Biophys. Acta,* 2001, vol. 1525 (1-2), 149-160